(19) Europäisches Patentamt
European Patent Office
Office européen des brevets

(11) Veröffentlichungsnummer: **0 000 333**
**B1**

(12)

# EUROPÄISCHE PATENTSCHRIFT

(45) Veröffentlichungstag der Patentschrift: **28.07.82**

(21) Anmeldenummer: **78100174.8**

(22) Anmeldetag: **16.06.78**

(51) Int. Cl.³: **C 07 D 211/14,**
C 07 D 211/22,
C 07 D 211/46,
A 01 N 43/40

(54) **Derivate cyclischer Amine und diese enthaltende Fungizide.**

(30) Priorität: **18.06.77 DE 2727482**

(43) Veröffentlichungstag der Anmeldung:
**24.01.79 Patentblatt 79/2**

(45) Bekanntmachung des Hinweises auf die Patenterteilung:
**28.07.82 Patentblatt 82/30**

(84) Benannte Vertragsstaaten:
**BE CH DE FR GB LU NL SE**

(56) Entgegenhaltungen:
**DE - A - 2 752 096**
**DE - A - 2 752 135**
**GB - A - 809 760**
**GB - A - 1 409 438**
**NL - A - 77 13685**
**US - A - 4 008 276**

(73) Patentinhaber: **BASF Aktiengesellschaft**
**Carl-Bosch-Strasse 38**
**D-6700 Ludwigshafen (DE)**

(72) Erfinder: **Himmele, Walter, Dr.**
**Eichenweg 14**
**D-6909 Walldorf (DE)**
Erfinder: **Pommer, Ernst-Heinrich, Dr.**
**Berliner Platz 7**
**D-6703 Limburgerhof (DE)**
Erfinder: **Goetz, Norbert, Dr.**
**Schoefferstrasse 25**
**D-6520 Worms (DE)**
Erfinder: **Zeeh, Bernd, Dr.**
**Thorwaldsenstrasse 5**
**D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 333 B1

## Derivate cyclischer Amine und diese enthaltende Fungizide

Die vorliegende Erfindung betrifft neue wertvolle Derivate cyclischer Amine und ihre Salze und Molekül- und Additionsverbindungen mit guter fungizider Wirkung, Fungizide, die diese Verbindungen enthalten, und Verfahren zur Bekämpfung von Pilzen mit diesen Verbindungen.

Es ist bekannt, N-Tridecyl-2,6-dimethylmorpholin und seine Salze und seine Molekül- und Additionsverbindungen als Fungizide zu verwenden (DT—PS 11 64 152, DT—PS 11 73 722, DT—OS 24 61 513).

Es wurde gefunden, daß Derivate cyclischer Amine der Formel

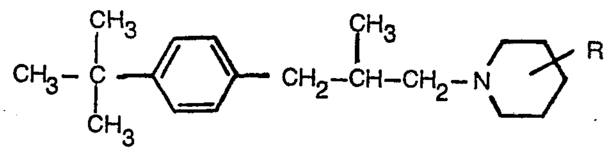

in der

R die Hydroxylgruppe, die Hydroxymethylgruppe, die Phenylgruppe oder die Reste $-O-CO-CH_3$ oder $O-CO-C_2H_5$ bedeutet und ihre Salze, Molekül- und Additionsverbindungen eine gute fungizide Wirkung haben, die der Wirkung der bekannten Morpholinderivate überlegen ist.

Salze sind beispielsweise die Salze mit anorganischen oder organischen Säuren, z.B. Chloride, Fluoride, Bromide, Jodide, Sulfate, Nitrate, Phosphate, Acetate, Propionate, Molekül- oder Additionsverbindungen entstehen beispielsweise mit Säuren von Tensiden, z.B. Dodecylbenzolsulfonsäure,

$$HO_3S-CH_2-CH_2-\overset{O}{\overset{\|}{C}}-(O-CH_2-CH_2)_2-OH \text{ oder } HO_3S-CH_2-CH_2-\overset{O}{\overset{\|}{C}}-(O-CH_2-CH_2)_3-OH.$$

Die Herstellung der neuen Verbindungen erfolgt beispielsweise durch Umsetzung von 3-Hydroxymethylpiperidin mit 3-p-Tertiär-butyl-phenyl-2-methyl-propanol in Gegenwart eines Redutionsmittels, z.B. Ameisensäure bei Temperaturen von 50 bis 110°C.

Von den erfindungsgemäßen Verbindungen werden beispielsweise die folgenden genannt:

R = 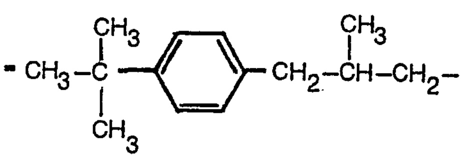

Wirkstoff Nr. 1 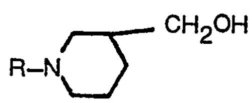 Kp: 195—204°/5,0 Torr

Wirkstoff Nr.

2 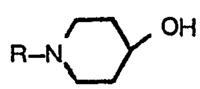 Kp: 200—205°/5,0 Torr

3 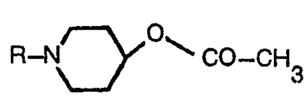 Kp: 185—190°/4,0 Torr

4 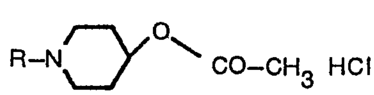 Fp: 210°

5 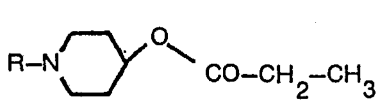 Kp: 160—170°/0,3 Torr

6 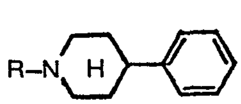 Kp: 210—222°/5,0 Torr

Die erfindungsgemäßen Wirkstoffe und die entsprechenden Fungizide sind insbesondere geeignet zur Bekämpfung von Pflanzenkrankheiten, z.B. Erysiphe graminis (echter Mehltau) an Getriede, Erysiphe cichloriacearum (echter Mehltau) an Kürbisgewächsen, Podosphaera leucotricha an Äpfeln, Uncinula necator an Reben, Erysiphe polygoni an Bohnen. Sphaerotheca pannosa an Rosen, Microsphaera querci an Eichen, Botrytis cinerea an Erdbeeren, Reben, Mycosphaerella musicola an Bananen, Puccinia-Arten (Rostpilze) an Getreide, Uromyces appendiculatus und U. phaseoli an Bohnen, Hemileia vastatrix an Kaffee und Rhizoctonia solani. Sie sind systematisch wirksam; sie werden sowohl über die Wurzeln als auch über die Blätter aufgenommen und im Pflanzengewebe transportiert.

Bei der Anwendung der neuen Wirkstoffe zur Behandlung von Pflanzen gegen Pilzinfektionen liegen die Aufwandmengen zwischen 0,025 und 5 kg Wirkstoff/ha Fläche. Zum Oberflächenschutz von Bäumen oder Früchten können die Wirkstoffe auch in Verbindung mit Kunststoffdispersionen 0,25 prozentig, bezogen auf das Gewicht der Dispersion, verwendet werden. Die Fungizide enthalten im allgemeinen zwischen 0,1 und 95 Gewichtsprozent Wirkstoff, vorzugsweise zwischen 0,5 und 90%.

Die Wirkstoffe können mit anderen bekannten Fungiziden gemischt werden. In vielen Fällen erhält man dabei eine Vergrößerung des fungiziden Wirkungssepektrums; bei einer Anzahl von Fungizidmischungen in den Gewichtsverhältnissen 1 : 10 bis 10 : 1 treten auch synergistische Effekte auf, d.h., die fungizide Wirksamkeit des Kombinationsproduktes ist größer als die der addierten Wirksamkeiten der Einzelkomponenten. Fungizide, die mit den erfindungsgemäßen Verbindungen kombiniert werden können, sind beispielsweise:

Dithiocarbamate und deren Derivate, wie Zinkdimethyldithiocarbamat,
Manganäthylenbisdithiocarbamat,
Mangan-Zink-äthylendiamin-bis-dithiocarbamat,
Zinkäthylenbisdithiocarbamat,
Tetramethylthiuramdisulfid,
Ammoniak-Komplez von Zink-(N,N-äthylen-bis-dithiocarbamat) und
N,N'-Polyäthylen-bis-(thiocarbamoyl)-difulsid,
Zink-(n,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;
heterocyclische Verbindungen, wie N-Trichlormethylthio-tetrahydrophthalimid,
N-Trichlormethylthio-phthalimid,
N-(1,1,2,2-Tetrachloräthylthio)-tetrahydrophthalimid,
1-(Butylcarbamoyl)-2-benzimidazol-carbaminsäuremethylester,
2-Methyloxycarbonylamino-benzimidazol,
2,3-Dihydro-5-carboxanilido-6-methyl-1,4-oxathiin-4,4-dioxid,
2,3-Dihydro-5-carboxanilido-6-methyl-1,4-oxathiin,
5-Butyl-2-dimethylamino-4-hydroxy-6-methyl-pyrimidin,
1,2-Bis-(3-äthoxycarbonyl-2-thioureido)-benzol,
1,2-Bis-(3-methoxycarbonyl-2-thioureido)-benzol
und verschiedene andere Fungizide, wie Dodecylguanidinacetat,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Jodbenzoesäureanilid,
2-Brombenzoesäureanilid,
3-Nitro-isophthalsäure-diisopropylester,
1-(1,2,4-Triazolyl-1')-1-(4'-chlorphenoxy)-3,3-dimethylbutan-2-on,
1-(1-Imidazolyl)-2-allyloxy-2-(2,4-dichlorphenyl)-äthan,
Piperazin-1,4-diyl-bis-1-(2,2,2-Trichloräthyl)-formamid,
2,4,5,6-Tetrachloro-isophthalonitril,
1,2-Dimethyl-3,5-diphenyl-pyrazoliniummethylsulfat.

Die Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen, Beizen oder Gießen. Die Anwendungsformen richten sich ganz nach den Verwendungszwecken; sie sollten in jedem Fall möglichst die feinste Verteilung der erfindungsgemäßen Wirkstoffen gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:
Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formealdehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitblaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kalk, Kreide, Talkum, Bolus, Löß, Ton, Dolomit, Diatemeenerde, Calcium- und Magnesiumsulfat, Magnesiumoxid, gemahlene Kunststoffe, Düngemittel, wie z.B. Ammoniumsulfate, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Zu den Mischungen oder Einzelwirkstoffen können Öle verschiedenen Typs, Herbizide, Fungizide, Nemtatozide, Insektizide, Bakterizide, Spurenelemente, Düngemittel, Antischaummittel (z.B. Silikone), Wachstumsregulatoren, Antidotmittel oder andere wirksame Verbindungen, zugesetzt werden.

Für die folgenden Versuche wurden folgende bekannte Vergleichssubstanzen verwendet.

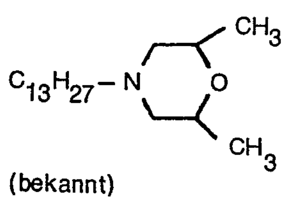

A

(bekannt)

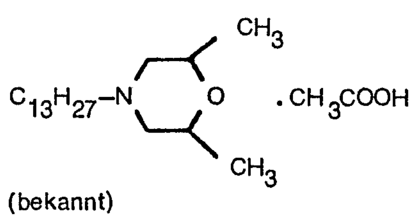

B

(bekannt)

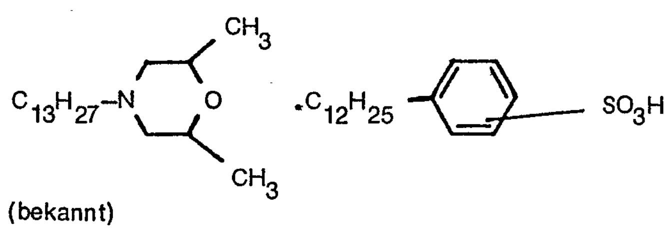

C

(bekannt)

Beispiel 1

Blätter von in Töpfen gewachsenen Weizenkeimlingen der Sorte "Jubilar" werden mit wäßrigen Emulsionen aus 80% (Gewichtsprozent) Wirkstoff und 20% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewäschshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

| Wirkstoff Nr. | Befall der Blätter nach Spritzungen mit x %iger Wirkstoffbrühe x=0,012 | 0,025 | 0,05 |
|---|---|---|---|
| 5 | 0 | 0 | 0 |

| Wirkstoff Nr. | Befall der Blätter nach Spritzungen mit x %iger Wirkstoffbrühe x= | 0,012 | 0,025 |
|---|---|---|---|
| A bekannt | 3 | 2 | 1 |
| B bekannt | 4 | 2 | 1 |
| C bekannt | 1 | 1 | 0 |
| Kontrolle (unbehandelt) | 4 | 4 | 4 |

0 kein Befal, abgestuft bis 5 Totalbefall

Beispiel 2

In einem weiteren Versuch werden, wie in Beispiel 3 beschrieben, Blätter von in Töpfen gewachsenen Gerstenkeimlingen der Sorte "Firlbecks Union" behandelt und mit Sporen (Konidien) des Gerstenmehltaus (Erysiphe graminis var. hordei) bestäubt.

| Wirkstoff Nr. | Befall der Blätter nach Spritzungen mit x %iger Wirkstoffbrühe x= 0,012 | 0,025 |
|---|---|---|
| 6 | 0 | 0 |

| Wirkstoff Nr. | Befall der Blätter nach Spritzungen mit x %iger Wirkstoffbrühe x= 0,012 | 0,025 |
|---|---|---|
| A bekannt | 1 | 0—1 |
| B bekannt | 1 | 1 |
| Kontrolle (unbehandelt) | 4 | 4 |

0 = kein Befall, abgestuft bis 5 = Totalbefall

Beispiel 3

Blätter von in Töpfen gewachsenen Weizenpflanzen werden mit Sporen des Weisenbraunrostes (Puccinia recondita) künstlich infiziert und 48 Stunden lang bei 20 bis 25°C in einer wasserdampfgesättigten Kammer aufgestellt. Danach werden die Pflanzen mit wäßrigen Spritzbrühen, die in dem Wasser gelöst oder emulgiert eine Mischung aus 80% des zu prüfenden Wirkstoffes und 20% Natriumligninsulfonat enthalten, besprüht und im Gewächshaus bei Temperaturen zwischen 20 und 22°C und bei 75 bis 80% relativer Luftfeuchtigkeit aufgestellt. Nach 10 Tagen wird das Ausmaß der Rostpilzentwicklung beurteilt.

In gleicher Weise werden Blätter von Gerstenpflanzen mit Sporen des Zwergrostes (Puccinia hordei) und Blätter von Haferpflanzen mit Sporen des Kronenrostes (Puccinia coronata) künstlich infiziert und mit den zu prüfenden Wirkstoffen behandelt.

| Wirkstoff Nr. | Befall der Blätter nach Spritzung mit 0,05%iger Wirkstoffbrühe | | |
|---|---|---|---|
| | Weizenbraunrost | Gerstenzwergrost | Haferkronenrost |
| 1 | 0 | 0 | 0 |
| 2 | 0 | 0 | 2 |
| 3 | 0 | 0 | 0 |
| 4 | 0 | 0 | 0 |
| 5 | 0 | 0 | 0 |
| 6 | 0 | 0 | 2 |

| Wirkstoff Nr. | Befall der Blätter nach Spritzung mit 0,05 %iger Wirkstoffbrühe | | |
|---|---|---|---|
| | Weizenbraunrost | Gerstenzwergrost | Haferkronenrost |
| A (bekannt) | 3 | 3 | 3 |
| B (bekannt) | 4 | 4 | 4 |
| C (bekannt) | 3 | 3 | 3 |
| Kontrolle (unbehandelt) | 4 | 4 | 4 |

0 = kein Befall, abgestuft bis 5 = Totalbefall

Beispiel 4

Man vermischt 90 Gewichtsteile der Verbindung 3 mit 10 Gewichtsteilen N-Methyl-$\alpha$-pyrrolidon und erhält eine Lösung, die zur Anwendung in Form kleinster Tropen geeignet ist.

Beispiel 5

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-mono-äthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 6

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 7

20 Gewichtsteile der Verbindung 2 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man ein wäßrige Dispersion, die 0,02 Gewichtsprozent des Wirkstoffs enthält.

Beispiel 8

20 Gewichtsteile des Wirkstoffs 3 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulferförmigem Kieselsäuregel gut vermischt und in einer Hammermühle vermahlen. Durch feines Verteilen der Mischung in 20 000 Gewichtsteilen Wasser erhält man eine Spritzbrühe die 0,1 Gewichtsprozent des Wirkstoffs enthält.

Beispiel 9

3 Gewichtsteile der Verbindung 3 werden mit 57 Gewichtsteilen feinteiligem Kaolin innig vermischt. Man erhält auf diese Weise ein Stäubemittel, das 3 Gewichtsprozent des Wirkstoffs enthält.

Beispiel 10

30 Gewichtsteile der Verbindung 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 11

40 Gewichtsteile des Wirkstoffs 3 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 12

20 Teile des Wirkstoffs 5 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-kondensats und 68 Teile eines paraffinischen Mineralöls innig vermischt. Man erhält eine stabile ölige Dispersion.

**Patentansprüche**

1. Derivate cyclischer Amine der Formel

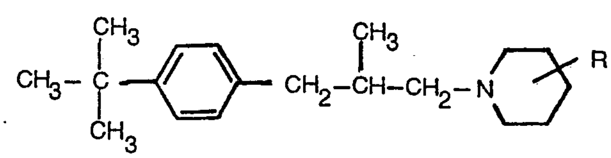

in der R die Hydroxylgruppe, die Hydroxymethylgruppe, die Phenylgruppe oder die Reste $-O-CO-CH_3$ oder $O-CO-C_2H_5$ bedeutet und ihre Salze, Molekül- und Additionsverbindungen.

2. Fungizid, enthaltend ein Derivat eines cyclischen Amins gemäß Anspruch 1.

**Claims**

1. Derivatives of cyclic amines of the formula

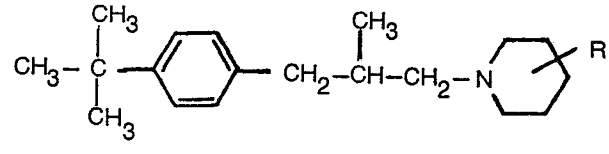

where R is hydroxyl, hydroxymethyl, pheny or $-O-CO-CH_3$ or $O-CO-C_2H_5$, and their salts, molecular compounds and adducts.

2. A fungicide containing a derivative of a cyclic amine as claimed in claim 1.

## Revendications

1. Dérivés d'amines cycliques de formule

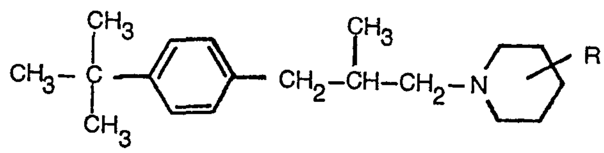

dans laquelle R désigne le groupe hydroxyle, le groupe hydroxyméthyle, le groupe phényle ou le reste $—O—CO—CH_3$ ou $—O—CO—C_2H_5$, et leurs sels, composés moléculaires et d'addition.

2. Fongicide, contenant un dérivé d'une amine cyclique selon la revendication 1.